(19) Europäisches Patentamt
European Patent Office
Office européen des brevets

(11) Veröffentlichungsnummer: **0 000 526**
**B1**

(12) # EUROPÄISCHE PATENTSCHRIFT

(21) Anmeldenummer: **78100407.2**

(22) Anmeldetag: **17.07.78**

(51) Int. Cl.³: **A 61 K 31/43,**
A 61 K 31/545, //
(A 61 K 31/43, 31/42).
(A 61 K 31/545, 31/42)

(54) **Pharmazeutische Präparate die Penicilline oder Cephalosporine und Clavulansäure enthalten, Verfahren zu deren Herstellung**

(30) Priorität: **27.07.77 DE 2733642**

(43) Veröffentlichungstag der Anmeldung:
**07.02.79 Patentblatt 79/03**

(45) Bekanntmachung des Hinweises auf die Patenterteilung:
**12.11.80 Patentblatt 80/23**

(84) Benannte Vertragsstaaten:
**BE CH DE FR GB LU NL SE**

(56) Entgegenhaltungen:
**DE - A - 2 648 770**

(73) Patentinhaber: **BAYER AG**
**ZENTRALBEREICH PATENTE, MARKEN UND LIZENZEN**
**D - 5090 LEVERKUSEN 1, BAYERWERK (DE)**

(72) Erfinder: **Metzger, Karl Georg, Dr.**
**Pahlkestrasse 15**
**D - 5600 Wuppertal 1 (DE)**
**König, Hans-Bodo Dr.**
**Herberts Katernberg 10**
**D - 5600 Wuppertal 1 (DE)**
**Schröck, Wilfried, Dr.**
**Pahlkestrasse 33**
**D - 5600 Wuppertal 1 (DE)**
**Preiss, Michael, Dr.**
**Egenstrasse 21**
**D - 5600 Wuppertal 1 (DE)**
**Feyen, Peter, Dr.**
**Mozartstrasse 1**
**D - 4020 Mettmann (DE)**

EP 0 000 526 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Pharmazeutische Präparate die Penicilline oder Cephalosporine und Clavulansäure enthalten, Verfahren zu deren Herstellung

Die vorliegende Erfindung betrifft neue antibiotische synergistische Wirkstoffkombinationen aus $\beta$ - Lactam - Antibiotika einerseits und der als Synergist wirkenden Clavulansäure oder ihren pharmazeutisch verwendbaren Salzen oder Estern, insbesondere ihrem Natriumsalz, andererseits.

Es ist bekannt (DT-OS 2 517 316), daß Clavulansäure auf $\beta$-Lactamasen eine Hemmwirkung auszuüben vermag. Es ist ferner bekannt und experimentell belegt, daß die minimalen Hemmkonzentrationen von Ampicillin, Amoxicillin, Carbenicillin und Benzylpenicillin durch Clavulansäure als Synergist erheblich vermindert werden (siehe DT-OS 2 517 316, Beispiel 42 und 44).

Aus den angegebenen Daten ist ersichtlich, daß im Durchschnitt die Mischung von Ampicillin mit Clavulansäure die beste antibiotische Wirksamkeit hat.

Es wurde nun gefunden, daß die neue Wirkstoffkombination aus (1) Penicillin oder Cephalosporinen der allgemeinen Formel:

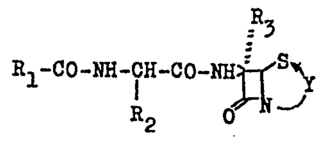

I

in welcher $R_1$ für

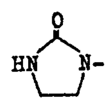 ,

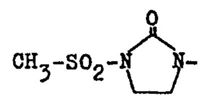 ,

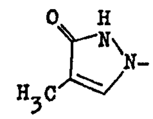 ,

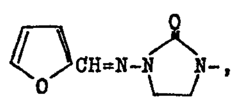 ,

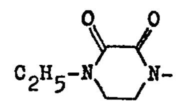

und

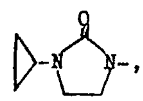 ,

$R_2$ für Phenyl, p-Hydroxyphenyl und Furyl,
$R_3$ für H, $OCH_3$ und $OC_2H_5$ steht, worin Y

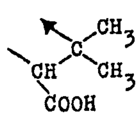

und

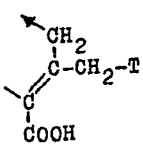

bedeutet, worin T für $—O—CO—CH_3$,

$—O—CO—NH_2$, 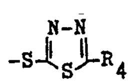 , 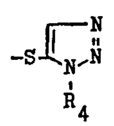 , 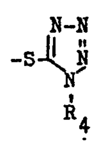 steht

und $R_4$ H, $CH_3$, $—CH_2—COOH$, $—CH_2—SO_3H$ oder $—CH_2—CH_2—N(CH_3)_2$ ist,

oder deren pharmazeutisch verwendbaren Salzen, insbesondere den Natriumsalzen, sowie deren verschiedenen Kristall- und Hydratformen und (2) Clavulansäure der Formel II:

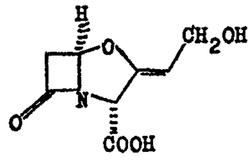

(II)

oder ihren pharmazeutisch verwendbaren Salzen oder in vivo spaltbaren Estern, insbesondere ihrem Natriumsalz eine besonders hohe antibiotische Wirksamkeit besitzen.

Beispiele für bevorzugt als Komponente (1) eingesetzte $\beta$-Lactam-Antibiotika der Formel I sind: D - $\alpha$[(Imidazolidin - 2 - oxo - 1 - yl) - carbonylamino] - benzyl - penicillin, D - $\alpha$[(3 - Methylsulfonyl - 2 - oxo - imidazolidin - 1 - yl) - carbonylamino] - benzylpenicillin, D - $\alpha$[(4 -

Methyl - 5 - oxo - 1,2 - diazol - 3 - in - 2 - yl) - carbonylamino] - benzylpenicillin, D - α[(3 - furfurylidenamino - 2 - oxo - imidazolidin - 1 - yl) - carbonyl-amino] - p-hydroxybenzylpenicillin, D - α[(4 - Äthyl - 2,3 - dioxo - piperazino - 1 - yl) - carbonylamino] - benzylpenicillin und D - α[(4 - Äthyl - 2,3 - dioxo - piperazino - 1 - yl) - carbonylamino] - p - hydroxy - benzyl - penicillin, D - 6 - β - { - 2[(4 - Äthyl - 2,4 - dioxo - piperazino - 1 - yl) · carbonylamino] - phenylacetamindo} - 6 - α - methoxypenicillansäure, D - 6 - β - {2[(3 - Furfurylidenamino - 2 - oxo - imidazolidin - 1 - yl) - carbonylamino] - p - hydroxyphenylacetamido} - 6 - α - methoxy - penicillansäure, D - 7 - β{ - 2[(3 - Furfurylidenamino - 2 - oxo - imidazolidin - 1 - yl) - carbonylamino] - fur - 2 - yl - acetamido} - 7 - α - methoxy-cephalosporansäure, D - 6 - β - { - 2[(4 - Äthyl - 2,3 - dioxo - piperazino - 1 - yl) - carbonylamino] - p - hydroxyphenylacetamido} - 6 - α - methoxy - penicillansäure, D - 7 - β { - 2[(3 - Furfurylidenamino - 2 - oxo - imidazolidin - 1 - yl) - carbonylamino] - phenylacetamido} - 7 - α - methoxy-cephalosporansäure und D - 7 - β { - 2[(3 - Cyclopropyl -2 - oxo - imidazolidin - 1 - yl) - carbonylamino] - phenylacetamido} - 7 - α - methoxy - cephalosphoransäure.

Die genannten β-Lactam-Antibiotika sowie ihre pharmazeutisch verwendbaren Salze sind bekannt [s. z.B. DT-OS 2 104 580, DT-OS 2 152 967, DT-OS 2 525 541, DT-OS 2 525 541, 2 519 400, US-PS 3 974 288].

Clavulansäure, sowie deren pharmazeutisch verwendbaren Salze und in vivo spaltbaren Ester werden in der DT-OS 2 517 316 beschrieben.

Beispielsweise wurde gefunden, daß die erfindungsgemäße Wirkstoffkombination aus D - α[(3 - Methylsulfonyl - 2 - oxo - imidazolidin - 1 - yl) - carbonylamino] - benzylpenicillin mit Clavulansäure bezüglich ihrer antibiotischen Wirksamkeit einer Wirkstoffkombination aus Ampicillin und Clavulansäure, wie sie in der DT-OS 2 517 316 als besonders wirksam beschrieben ist, überraschend stark überlegen ist, und zwar ist sie gegen E.coli F14, E.coli Münster, Klebsiella 1857 und Proteus morg. 932, etwa 3-mal besser, gegen E.coli T7, E.coli A 261 und Proteus vulg. 1017 etwa 6-mal besser, gegen Klebsiella 57 USA etwa 12-mal besser und gegen Proteus morg. 1102/III etwa 190-mal besser als die Wirkstoffkombination der Offenlegungsschrift Nr. 2 517 316, bestehend aus Ampicillin und Clavulansäure.

Es muß weiterhin überraschen, daß die antibakterielle Wirksamkeit der erfindungsgemäßen Wirkstoffkombination wesentlich höher ist als die Summe der Wirkungen der einzelnen Wirkstoffe. Es liegt also ein echter synergistischer Effekt vor. Die Wirkstoffkombinationen stellen somit eine wertvolle Bereicherung der Pharmazie dar.

Die Gewichtsverhältnisse der Wirkstoffe (1) und (2) können in relativ großen Bereichen schwanken, und zwar vom Verhältnis (1) zu (2) wie 1 zu 10 bis zum Verhältnis (1) zu (2) wie 10 zu 1. Bevorzugt ist der Bereich (1) zu (2) wie 1 zu 5 bis 5 zu 1, ganz besonders bevorzugt der Bereich 1 zu 3 bis 3 zu 1.

Gegenstand der Erfindung sind ferner Arzneimittel mit einem Gehalt an einer Wirkstoffkombination, bestehend aus einem Penicillin oder Cephalosporin der allgemeinen Formel I bzw. einem pharmazeutisch verwendbarem Salz davon und Clavulansäure bzw. ihren pharmazeutisch verwendbaren Salzen oder Estern. Bevorzugt sind die Natriumsalze. Bei den Estern der Clavulansäure sind die Methyl- und Äthylester bevorzugt.

Die vorliegende Erfindung umfaßt solche Arzneimittelformulierungen, die parenteral, lokal oder oral angewendet werden können. Bevorzugt sind parenterale und orale, ganz besonders parenterale Anwendungsformen. Demgemäß umfassen die Anwendungsformen bevorzugt sterile, zu Injektionsoder Infusionszwecken geeignete Formulierungen.

In einer formulierten Einzeldosis pro Mensch können Mischungen von Clavulansäure oder ihren Salzen oder Estern mit den genannten Penicillin oder Cephalosporinen, beispielsweise D - α[(3 - Methylsulfonyl - 2 - oxo - imidazolidin - 1 - yl) - carbonylamino] - benzylpenicillin - Natrium, mit einer Gesamtmenge von 0,5 bis 10 g vorliegen. An einem Behandlungstag pro Mensch können etwa 0,5 g bis 30 g des Wirkstoffgemisches verabreicht werden. Dieses sind jedoch keine beschränkenden Angaben; denn die Einzeldosis und die Dosierungsintervalle variieren sehr mit der Schwere der Infektion.

In den erfindungsgemäßen Arzneimitteln können neben der Clavulansäure bzw. ihren Estern oder Salzen, z.B. ihrem Natriumsalz, auch mehrere der genannten Penicilline bzw. Cephalosporine, z.B. D - α - [(3 - Methylsulfonyl - 2 - oxo - imidazolidin - 1 - yl) - carbonylamino] - benzylpenicillin - Natrium und D - α - [(Imidazolidin - 2 - oxo - 1 - yl) - carbonylamino] - benzylpenicillin. Bevorzugt sind jedoch solche Arzneimittel, die neben Clavulansäure bzw. ihren Estern oder Salzen nur ein Penicillin enthalten.

Die erfindungsgemäßen Arzneimittel weisen bei guter Verträglichkeit eine starke antimikrobielle Wirksamkeit auf.

Die erfindungsgemäßen Arzneimittel sind gegen ein sehr breites Spektrum von Mikroorganismen wirksam. Mit ihrer Hilfe können z.B. gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Wirkstoffgemische gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis (Staph. = Staphylococcus);

Lactobacteriaceae, wie Streptokokken, z.B. Streptococcus pyogenes, $\alpha$- bzw. $\beta$- hämolysierende Streptokokken, nicht ($\gamma$)-hämolysierende Streptokokken, Str. viridans, Str. faecalis (Enterokokken) und Diplococcus pneumoniae (Pneumokokken) (Str. = Streptococcus);

Neisseriaceae, wie Neisserien, z.B. N.meningitidis (Meningokokken), N.catarrhalis (N. = Neisseria);

Corynebacteriaceae, wie Corynebakterien, z.B. Corynebacterium diphtheriae, C. pyogenes, C.aones (C. = Corynebacterium);

Enterobacteriaceae, wie Escherichiae-Bakterien der Coli-Gruppe, Escherichia-Bakterien, z.B. Escherichia coli, Enterobacter-Bakterien, z.B. E. aerogenes, E. cloacae, Klebsiella-Bakterien, z.B. K. pneumoniae, Serratia, z.B. Serratia marcescens (E. = Enterobacter) (K. = Klebsiella), Proteae-Bakterien der Proteus-Gruppe, Proteus, z.B. Proteus vulgaris, Pr. morganii, Pr. rettgeri, Pr. mirabilis (Pr. = Proteus), Providencia z.B. Providencia sp., Salmonelleae, Salmonella-Bakterien, z.B. Salmonella paratyphi A und B, S. typhi, S. enteritidis, S. cholerae suis, S. Typhimurium (S. = Salmonella), Shigella-Bakterien z.B. Shigella dysenteriae, Sh. sonnei (Sh. = Shigella);

Pseudomonadaceae, wie Pseudomonas-Bakterien, z.B. Pseudomonas aeruginosa, Aeromonas-Bakterien, z.B. Aeromonas liquefaciens, A. Hydrophila (A. = Aeromonas);

Parvobacteriaceae oder Brucellaceae, wie Pasteurella-Bakterien, z.B. Pasteurella multocida, Past. pseudotuberculosis (Past. = Pasteurella), Haemophilus-Bakterien, z.B. Haemophilus influenzae.

Bacteroidacea, wie Bacteroides-Bakterien, z.B. Bacteroides fragilis.

Bacillaceae, wie aerobe Sporenbildner, z.B. Bacillus anthracis, B.subtilis, B.cereus (B. = Bacillus), anaerobe Sporenbildner-Chlostridien, z.B. Clostridium perfringens, Cl.tetani (Cl. = Chlostridium).

Die obige Aufzählung von Erregern ist lediglich beispielhaft und keineswegs beschränkend aufzufassen.

Als Krankheiten, die durch die erfindungsgemäßen Wirkstoffkombinationen verhindert, gebessert und/oder geheilt werden können, seien beispielsweise genannt.

Erkrankungen der Atmungswege und des Rachenraumes; Otitis; Pharyngitis; Pneumonie; Peritonitis; Pyelonephritis; Cystitis; Endocarditis; Systeminfektion; Bronchitis; Arthritis.

Zur vorliegenden Erfindung gehören pharmazeutische Zubereitungen, die neben nichttoxischen, inerten pharmazeutisch geeigneten Trägerstoffen Clavulansäure und ein oder mehrere erfindungsgemäße Penicilline bzw. Cephalosporine sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile. z.B. Tabletten, Dragees, Kapseln, Pillen, Suppositorien und Ampullen vorliegen, deren Wirkstoffgemisch einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis erhält vorzugsweise die Menge Wirkstoff, die bei einer Applikation verabreicht wird und die gewöhnlich einer ganzen, einer halben oder einem Drittel oder Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten. Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Ueberzügen und Hüllen versehen sein und auch so zusammengesetzt sein, daß sie den oder die Wirkstoffe nur oder bevorzugt in einem bestimmten Teil des Intestinaltraktes, gegebenenfalls verzögert, abgeben, wobei als Einbettungsmassen z.B. Polymersubstanzen und Wachse verwendet werden können.

Die erfindungsgemäßen Wirkstoffgemische können gegebenenfalls mit einem oder mehreren der oben angegebenen Trägerstoffe auch in mikroverkapselter Form vorliegen.

Suppositorien können neben dem Wirkstoffgemische die üblichen wasserlöslichen oder wasserunlöslichen Trägerstoffe enthalten, z.B. Polyäthylenglykole, Fette, z.B. Kakaofett, und höhere Ester (z.B. $C_{14}$-Alkohol mit $C_{16}$-Fettsäure) und Gemische dieser Stoffe.

Salben, Pasten, Cremes und Gele können neben dem Wirkstoffgemisch die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem Wirkstoffgemisch die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem Wirkstoffgemisch die üblichen Trägerstoffe wie Lösungsmittel, Lösungsvermittler und Emulgatoren, z.B. Wasser, Aethylalkohol, Isopropylalkohol, Aethylcarbonat, Aethylacetat, Benzylalkohol, Benzylbenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem Wirkstoffgemisch die üblichen Trägerstoffe wie flüssige Verdünnungsmittel, z.B. Wasser, Aethylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische oder Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl, und Süßmittel z.B. Saccharin enthalten.

Die therapeutisch wirksamen Wirkstoffkombinationen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die Herstellung der oben aufgeführten pharmazeutischen Zubereitungen erfolgt in üblicher Weise nach bekannten Methoden, z.B. durch Mischen des Wirkstoffgemisches mit pharmazeutisch verwendbaren Träger- und Zusatzstoffen.

Die Wirkstoffgemische oder die pharmazeutischen Zubereitungen können lokal, oral, parenteral, intraperitoneal und/oder rectal, vorzugsweise parenteral, insbesondere intravenös und intramusculös appliziert werden.

Im allgemeinen hat es sich sowohl in der Human- als auch in der Veterinärmedizin als vorteilhaft erwiesen, das erfindungsgemäße Wirkstoffgemisch in Gesamtmengen von etwa 1,5 bis etwa 60, vorzugsweise 1,5 bis 15 g/Mensch je 24 Stunden, gegebenenfalls in Form mehrerer, z.B. von 3 Einzelgaben, zur Erzielung der gewünschten Ergebnisse zu verabreichen. Eine Einzelgabe enthält den oder die erfindungsgemäßen Wirkstoffgemische vorzugsweise in Mengen von etwa 0,1 bis etwa 20 g, insbesondere 0,1 bis 1,5 g/Mensch. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoffgemisch auszukommen, während in anderen Fällen die oben angeführte Wirkstoffmenge überschritten werden muß. Die Festlegung der jeweils erforderlichen optimalen Dosierung und Applikationsart des Wirkstoffgemisches kann durch jeden Fachmann aufgrund seines Fachwissens leicht erfolgen.

Im Falle der Anwendung als Futterzusatzmittel können die neuen Wirkstoffgemische in üblicher Weise zusammen mit dem Futter bzw. mit Futterzubereitungen oder mit dem Trinkwasser gegeben werden. Dadurch kann eine Infektion durch gramnegative oder grampositive Bakterien verhindert und ebenso eine bessere Verwertung des Futters erreicht werden.

Die neuen Wirkstoffkombinationen zeichnen sich durch starke antibakterielle Wirkungen, die in vivo und in vitro geprüft wurden, und durch orale Resorbierbarkeit aus.

Die Wirksamkeit der erfindungsgemaßen Wirkstoffkombination kann beispielhaft durch den folgenden in vitro-Versuch demonstriert werden:

## Beispiel

Vergleich der MHK der erfindungsgemäßen Wirkstoffkombination aus dem Penicillin D - $\alpha$[(3 - Methylsulfonyl - 2 - oxo - imidazolidin - 1 - yl) - carbonylamino] - benzylpenicillin - Natrium und dem Natriumsalz der Clavulansäure (Kombination A) mit de MHK der Wirkstoffkombination aus den Natriumsalzen von Ampicillin und Clavulansäure (Kombination B) gemäß Offenlegungsschrift Nr. 2 517 316.

Methode: Es wurden die minimalen Hemmkonzentrationen im Röhrchenverdünnungstest bestimmt. Ablesung erfolgte nach 24 Stunden. Bei beiden Kombinationen (A und B) wurden jeweils gleiche Gewichtsmengen des Penicillins und der Clavulansäure miteinander kombiniert. Die Zahlen in

der folgenden Tabelle geben die niedrigste noch wirksame Menge des jeweiligen Penicillins (in der Kombination A bzw. B) an, und zwar in E/ml, die noch gegen das jeweils angegebene Bakterium wirksam war. In der Spalte F ist angegeben, um wievielmal die erfindungsgemäße Wirkstoffkombination A bei den einzelnen Keimen jeweils besser ist, als die zum Stand der Technik gehörende Kombination B.

TABELLE

M H K

| Keim | Kombination A E/ml: (Penicillin) | Kombination B E/ml: (Penicillin) | F (ca.) |
|---|---|---|---|
| E. coli T 7 | 2,1 | 12,8 | 6,1 |
| E. coli A 261 | 1,1 | 6,4 | 5,8 |
| E. coli F 14 | 4,2 | 12,8 | 3,0 |
| E. coli Münster | 1,1 | 3,2 | 2,9 |
| Klebs. 57 USA | 1,1 | 12,8 | 11,6 |
| Klebs. 1857 | 4,2 | 12,8 | 3,0 |
| Prot. morg. 932 | 17,0 | 49,7 | 2,9 |
| Prot. vulg. 1017 | 1,1 | 6,4 | 5,8 |
| Prot. morg. 1102/III | <0,53 | 100,3 | 189 |

Methode: Bestimmung der minimalen Hemmkonzentration im Röhrchenverdünnungstest, Ablesung nach 24 Stunden; es wurden gleiche Gewichtsmengen kombiniert; die MHK-Zahlen in der Tabelle geben die niedrigste wirksame Menge an.

**Patentansprüche**

1. Antibiotisches Mittel, gekennzeichnet durch einen Gehalt an einer Wirkstoffkombination bestehend aus (1) β-Lactam-Antibiotika der Formel I:

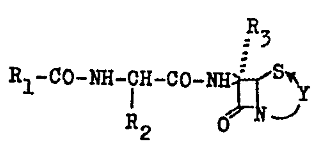

I

in welcher $R_1$ für 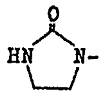 , 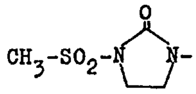 , 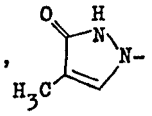 ,

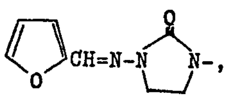 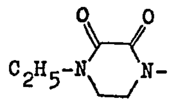 und 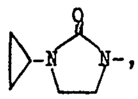

$R_2$ für Phenyl, p-Hydroxyphenyl und Furyl, $R_3$ für H, $OCH_3$ und $OC_2H_5$ steht, worin Y

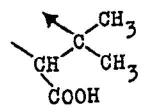 und 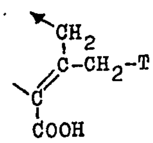

bedeutet, worin T für $—O—CO—CH_3$,

$—O—CO—NH_2$, 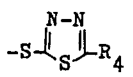 , 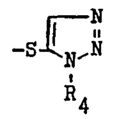 , 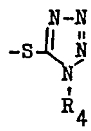 steht

und $R_4$ H, $CH_2$, $—CH_2COOH$, $—CH_2—SO_3H$ oder $—CH_2—CH_2—N(CH_3)_2$ ist, oder deren pharmazeutisch verwendbaren Salzen, insbesondere den Natriumsalzen, sowie deren verschiedenen Kristall- und Hydratformen und (2) Clavulansäure der Formel II:

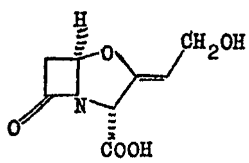

(II)

oder ihren phamazeutisch verwendbaren Salzen oder in vivo spaltbaren Estern, insbesondere ihrem Natriumsalz.

2. Antibiotiches Mittel gemäß Anspruch 1, das ein β-Lactam-Antibiotikum aus der Gruppe D - α[(Imidazolidin - 2 - oxo - 1 - yl) - carbonylamino] - benzyl - penicillin, D - α[(3 - Methylsulfonyl - 2 - oxo - imidazolidin - 1 - yl) - carbonylamino] - benzylpenicillin, D - α[(4 - Methyl - 5 - oxo - 1,2 - diazol - 3 - in - 2 - yl) - carbonylamino] - benzylpenicillin, D - α[(3 - furfurylidenamino - 2 - oxo - imidazolidin - 1 - yl) - carbonyl-amino] - p-hydroxybenzylpenicillin, D - α[(4 - Äthyl - 2,3 - dioxo - piperazino - 1 - yl) - carbonylamino] - benzylpenicillin und D - α[(4 - Äthyl - 2,3 - dioxo - piperazino - 1 - yl) - carbonylamino] - p - hydroxy - benzyl - penicillin, D - 6 - β - { - 2[(4 - Äthyl - 2,4 - dioxo - piperazino - 1 - yl) - carbonylamino] - phenylacetamindo} - 6 - α - methoxypenicillansäure, D - 6 - β - {2[(3 - Furfurylidenamino - 2 - oxo - imidazolidin - 1 - yl) - carbonylamino] - p - hydroxyphenylacetamido} - 6 - α - methoxy - penicillansäure, D - 7 - β{ - 2[(3 - Furfurylidenamino - 2 - oxo - imidazolidin - 1 - yl) - carbonylamino] - fur - 2 - yl - acetamido} - 7 - α - methoxy-cephalosporansäure, D - 6 - β - { - 2[(4 - Äthyl - 2,3 - dioxo - piperazino - 1 - yl) - carbonylamino] - p - hydroxyphenylacetamido} - 6 - α - methoxy - penicillansäure, D - 7 - β{ - 2[(3 - Furfurylidenamino - 2 - oxo - imidazolidin - 1 - yl) - carbonylamino] - phenylacetamido} - 7 - α - methoxy - cephalosporansäure und/oder D - 7 - β{ - 2[(3 - Cyclopropyl - 2 - oxo - imidazolidin - 1 - yl) - carbonylamino] - phenylacetamido} - 7 - α - methoxy - cephalosphoransäure oder ein pharmazeutisch verwendbares Salz, insbesondere das Natriumsalz, der genannten Verbindungen enthält.

3. Antibiotisches Mittels gemäß den Ansprüchen 1 und 2, dadurch gekennzeichnet, daß in der Wirkstoffkombination das Gewichtsverhältnis von (1) zu (2) zwischen 1:10 und 10:1, vorzugsweise zwischen 1:5 und 5:1, liegt.

4. Antibiotisches Mittel gemäß Anspruch 1 zur Anwendung bei der Bekämpfung von durch Bakterien hervorgerufenen Erkrankungen.

**Revendications**

1. Agent antibiotique, caractérisé par une teneur en une combinaison de substances actives consistant en:
(1) des $\beta$-lactame-antibiotiques de formule I:

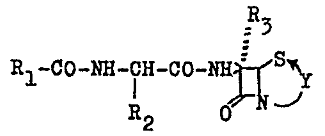

I

dans laquelle $R_1$ représente

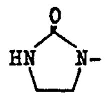 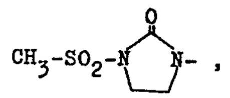 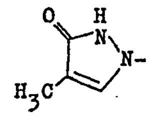

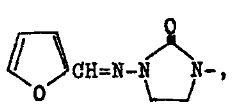 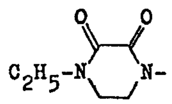 et 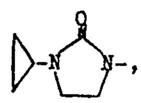

$R_2$ représente un phényle, p-hydroxyphényle et furyle,
$R_3$ représente H, $OCH_3$ et $OC_2H_5$, où Y représente

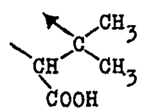 et 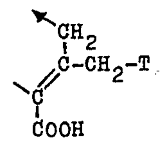

T représente $-O-CO-NH_3$, $-O-CO-NH_2$,

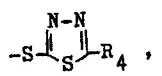 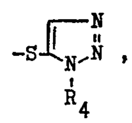 où 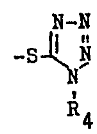 et

$R_4$ représente H, $CH_3$, $-CH_2-COOH$, $-CH_2-SO_3H$ ou $-CH_2-CH_2-N(CH_3)_2$, ou en leurs sels pharmaceutiquement utilisables, en particulier leurs sels de sodium ainsi que leurs diverses formes cristallisées et hydratées, et en (2) de l'acide clavulanique de formule II

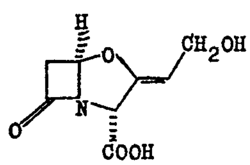

(II)

ou ses sels pharmaceutiquement utilisables ou esters clivables in vivo, en particulier son sel de sodium.

2. Agent antibiotique selon la revendication 1, caractérisé en ce qu'il contient un $\beta$-lactame-antibiotique du groupe:
D - $\alpha$[(imidazolidine - 2 - oxo - I - yl) - carbonylamino] - benzylpénicilline,
D - $\alpha$[(3-méthylsulfonyl - 2 - oxo - imidazolidine - 1 - yl) - carbonylamino] - benzylpénicilline,
D - $\alpha$[(4 - méthyl - 5 - oxo - 1,2 - diazol - 3 - ine - 2 - yl) - carbonylamino] - benzylpénicilline,
D - $\alpha$[(3 - furfurylidéneamino - 2 - oxo - imidazolidine - 1 - yl) - carbonylamino] - p - hydroxybenzylpénicilline,
D - $\alpha$[(4 - éthyl - 2,3 - dioxo - pipérazino - 1 - yl) - carbonylamino] - benzylpénicilline et
D - $\alpha$[(4 - éthyl - 2,3 - dioxo - pipérazino - 1 - yl) - carbonylamino] - p - hydroxybenzylpénicilline,

acide D - 6 - $\beta$ - {2[(4 - éthyl - 2,4 - dioxo - pipérazino - 1 - yl) - carbonylamino] - phénylacétamido} - 6 - $\alpha$ - méthoxypénicillanique,
acide D - 6 - $\beta$ - {2[(3 - furfurylidèneamino - 2 - oxo - imidazolidine - 1 - yl) - carbonylamino] - p - hydroxyphénylacétamido} - 6 - $\alpha$ - méthoxypénicillanique,
acide D - 7 - $\beta$ - {2[(3 - furfurylidèneamino - 2 - oxo - imidazolidine - 1 - yl) - carbonylamino] - fur - 2 - yl - acétamido} - 7 - $\alpha$ - méthoxycéphalosporanique.
acide D - 6 - $\beta$ - {2[(4 - éthyl - 2,3 - dioxo - pipérazino - 1 - yl) - carbonylamino] - p - hydroxyphénylacétamido} - 6 - $\alpha$ - méthoxypénicillanique,
acide D - 7 - $\beta$ - {2[(3 - furfurylidèneamino - 2 - oxo - imidazolidin - 1 - yl) - carbonylamino] - phénylacétamido} - 7 - $\alpha$ - méthoxycéphalosporanique et/ou
acide D - 7 - $\beta$ - {2[(3 - cyclopropyl - 2 - oxo - imidazolidine - 1 - yl) - carbonylamino] - phénylacétamido} - 7 - $\alpha$ - méthoxycéphalosproanique,
ou un sel pharmaceutiquement utilisable, en particulier le sel de sodium, des composés cités.

3. Agent antibiotique selon l'une quelconque des revendications 1 et 2, caractérisé en ce que dans la combinaison de substances actives le rapport pondéral de (1) à (2) est compris entre 1:10 et 10:1, de préférence entre 1:5 et 5:1.

4. Agent antibiotique selon la revendication 1 pour l'emploi en vue de combattre les maladies provoquées par des bactéries.

## Claims

1. An antibiotic composition, characterised in that it contains an active compound combination consisting of (1) $\beta$-lactam antibiotics of the formula I:

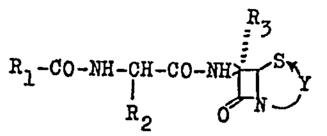

I

in which
R$_1$ represents 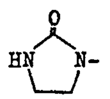 , 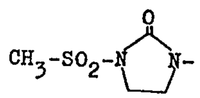 , 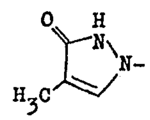 ,

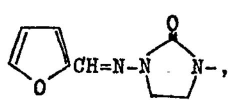 , 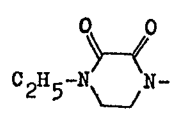 and 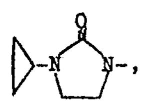 ,

R$_2$ represents phenyl, p-hydroxyphenyl or furyl,
R$_3$ represents H, $OCH_3$ or $OC_2H_5$
and wherein
Y denotes

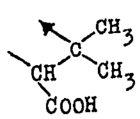 or 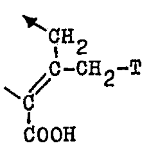 wherein

T represents $—O—CO—CH_3$, $—O—CO—NH_2$,

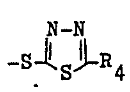 , 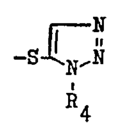 , or 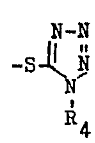 and

R$_4$ is H, $CH_3$, $—CH_2—COOH$, $—CH_2—SO_3H$ or $—CH_2—CH_2—N(CH_3)_2$
or their pharmaceutically usable salts, in particular the sodium salts, or their various crystal or hydrate forms and (2) clavulanic acid of the formula II

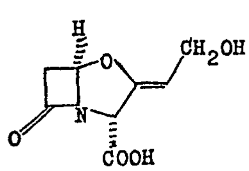

II

or its pharmacutically usable salts or esters which can be split vivo, in particular its sodium salt.

2. An antibiotic composition according to claim 1, which contains an $\beta$-lactam antibiotic from the group including D - $\alpha$ - [(imidazoliden - 2 - oxo - 1 - yl) - carbonylamino] - benzylpenicillin, D - $\alpha$ - [(3 - methylsulphonyl - 2 - oxo - imidazolidin - 1 - yl) - carbonylamino] - benzylpenicillin, D - $\alpha$ - [(4 - methyl - 5 - oxo - 1,2 - diazol - 3 - in - 2 - yl) - carbonylamino] - benzylpenicillin, D - $\alpha$ - [(3-furfurylideneamino - 2 - oxo - imidazolidin - 1 - yl) - carbonylamino] - p - hydroxybenzyl-penicillin, D - $\alpha$ - [(4 - ethyl - 2,3 - dioxo - piperazino - 1 - yl) - carbonylamino] - benzyl-penicillin and D - $\alpha$ - [(4 - ethyl - 2,3 - dioxo - piperazino - 1 - yl) - carbonylamino] - p - hydroxybenzyl - penicillin, D - 6 - $\beta$ - {2[(4 - ethyl - 2,4 - dioxo - piperazino - 1 - yl) - carbonyl-amino] - phenylacetamodo} - 6 - $\alpha$ - methoxy - penicillanic acid, D - 6 - $\beta$ - {2[(3 - furfuryl-ideneamino - 2 - oxo - imidazolidin - 1 - yl) - carbonylamino] - p - hydroxyphenylacetamido} - 6 - $\alpha$ - methoxy - penicillanic acid, D - 7 - $\beta$ - {2[(3 - furfurylideneamino - 2 - oxo - imidazolidin - 1 - yl) - carbonylamino] - fur - 2 - yl - acetamido} - 7 - $\alpha$ - methoxy - cephalosporanic acid, D - 6 - $\beta$ - {2[(4 - ethyl - 2,3 - dioxo - piperazino - 1 - yl) - carbonylamino] - p - hydroxy - phenyl - acetamido} - 6 - $\alpha$ - methoxy - penicillanic acid, D - 7 - $\beta$ - {2[(3 - furfurylideneamino - 2 - oxo - imidazolidin - 1 - yl) - carbonylamino] - phenylacetamido} - 7 - $\alpha$ - methoxy - imidazolidin - 1 - yl) - carbonylamino] - phenylacetamido} - 7 - $\alpha$ - methoxy - cephalosporanic acid and/or D - 7 - $\beta$ - {2[(3 - cyclopropyl - 2 - oxo - imidazolidin - 1 - yl) - carbonylamino] - phenylacetamido} - 7 - $\alpha$ - methoxy - cephalosporanic acid, or a pharmaceutically usable salt, in particular the sodium salt, of the named compounds.

3. An antibiotic composition according to claims 1 and 2, characterised in that the weight ratio of (1) to (2) in the active compound combination is between 1:10 and 10:1, preferably between 1:5 and 5:1.

4. An antibiotic composition according to claim 1 for use in combating diseases caused by bacteria.